# EUROPEAN PATENT SPECIFICATION

(11) **EP 1 140 288 B1**
(45) Date of publication and mention of the grant of the patent: **24.11.2004**
(21) Application number: 00901375.6
(22) Date of filing: 13.01.2000
(51) Int. Cl.: A61N 5/06

(54) **APPARATUS FOR ADMINISTERING EXTERNAL TREATMENT WITH THE AID OF LIGHT**
VORRICHTUNG ZUR VERABREICHUNG EINER EXTERNEN BEHANDLUNG MITTELS LICHT
DISPOSITIF PERMETTANT D'ADMINISTRER UN TRAITEMENT EXTERNE AU MOYEN DE LUMIERE

(30) Priority: 13.01.1999 SE 9900074
(43) Date of publication of application: 10.10.2001
(73) Proprietor: BIOLIGHT PATENT HOLDING AB, 182 33 Danderyd (SE)
(72) Inventor: THIBERG, Rolf, S-184 50 Akersberga (SE)
(74) Representative: Örtenblad, Bertil Tore
(86) International application number: PCT/SE2000/000044
(87) International publication number: WO 2000/041767

(56) References cited:
- WO-A1-97/46279
- US-A- 5 500 009

## Description

The present invention relates to an apparatus for external medical treatment with the aid of light, more specifically with the aid of light which palliates and/or cures different states of diseases.

Swedish Patent Specification No. 502 784 for example teaches an apparatus for external medical treatment with the aid of light that includes a light emitting device which is intended to be held against or close to the body of an individual, and drive means for operating the light emitting device, said device including light emitting diodes or corresponding elements which are intended to emit infrared light. According to the aforesaid patent specification, the means for driving the light emitting device is designed to control said device to emit infrared light in a first stage over a first predetermined time period and then to emit visible light in a second stage over a second predetermined time period, wherein said drive means is adapted to control the light emitting device to pulsate the infrared light and the visible light in accordance with a predetermined series of pulse frequencies.

It is also known to emit other monochromatic light for treating different states of diseases.

It has also been found that very good results can be,obtained when treating a patient with solely one or more types of monochromatic light and with light other than infrared light, such as visible light of different colours emitted in accordance with a given pulse frequency.

It has been found that a device of the aforesaid kind can be used very successfully for treating many different states of diseases, wounds and injuries, for instance sport sustained injuries, stretched muscles, muscular pain, joint pain, headaches, various inflammatory conditions, various skin complaints, such as acne, back pains, etc., provided that the light is emitted in a certain way. In this regard, treatment with light has a favourable influence on injury healing processes and will palliate and/or cure various diseases.

There is thus an understanding that treatment with certain light that is emitted in certain frequency series will have a significantly greater effect in shortening the time taken to cure or palliate a disease.

One problem with known apparatus of the present kind is that voltage is applied to the light emitting device, which is intended to be held against or in the close proximity of the patient's body, through a cable extending from a separate device in the form of a drive means which includes computer circuits and associated drive circuits for activating the light emitting diodes in said light emitting device. The apparatus is therefore relatively expensive and clumsy.

This problem is solved by the invention.

The present invention thus relates to an apparatus for controlling external medical treatment administered with the aid of light, said apparatus including a light-emitting device which is intended to be held against or in the close proximity of the patient's body, and means for driving the light-emitting device, wherein the light-emitting device includes light-emitting diodes adapted to emit monochromatic light, wherein the drive means is adapted to cause the light-emitting device to emit one or more types of monochromatic light over one or more predetermined time periods and to pulsate the light emitted in accordance with a predetermined pulse frequency or series of pulse frequencies over said time periods, wherein said drive means includes a computer and circuits for the light-emitting diodes, wherein the computer includes input means for inputting data relating to an intended treatment, wherein the computer is adapted to deliver electric signals to the drive circuits, wherewith intended light-emitting diodes function to emit light within predetermined time periods and at predetermined pulse repetition frequencies, and wherein the apparatus is characterised in that at least the drive circuits of the drive means are mounted in the light emitting device, in that the computer is adapted to send a coded signal to a microprocessor in the light emitting device via a conductor, wherewith the micro-processor functions to activate said drive circuits subsequent to decoding said signal and in that the computer is a personal computer and is free-standing in relation to the light emitting device.

The invention will now be described in more detail by way of example, partly in connecton with an embodiment of the invention shown in the accompanying drawings, in which
- Figure 1 is a schematic block diagram illustrating apparatus of the present kind;
- Figure 2 is a side view of a light emitting device;
- Figure 3 is a block diagram illustrating an apparatus according to a first embodiment of the invention;
- Figure 4 is a block diagram illustrating a second embodiment of the inventive apparatus; and
- Figure 5 illustrates one embodiment of the light emitting device of Figure 2 from above.

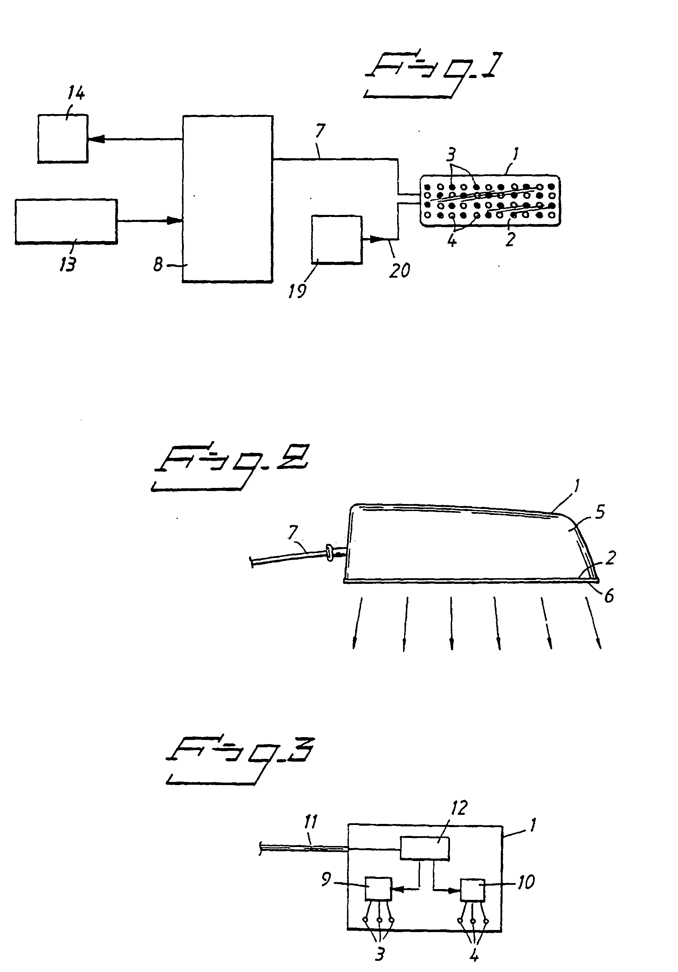

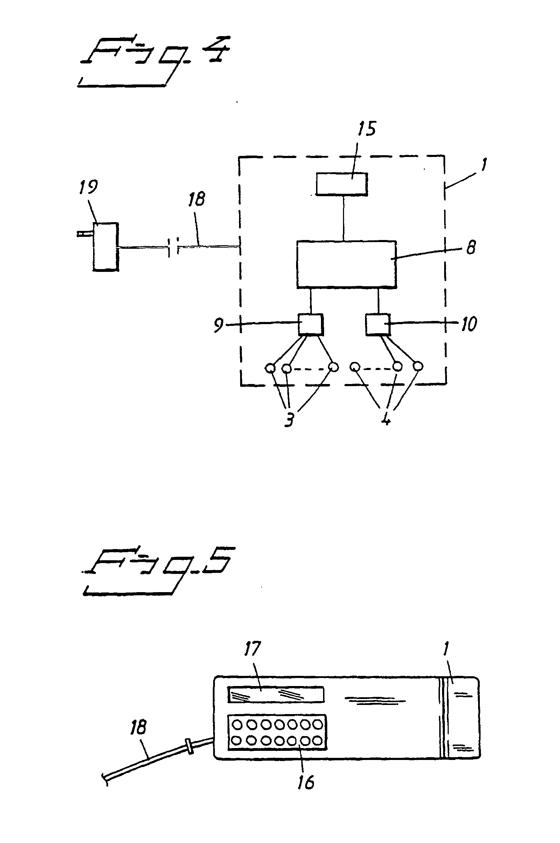

Figures 1 and 2 illustrate generally an apparatus for external medical treatment with the aid of light, said apparatus including a light emitting device 1 which is intended to be held against or in the close proximity of the patient's body. The light emitting device is shown from one side in Figure 2 and from beneath in Figure 1. This device includes a casing 5 which houses a transparent plate 6. Located beneath the plate 6 is a surface 2 on which a plurality of light emitting diodes 3, 4 or corresponding elements are mounted.

The light emitting diodes thus emit light through the plate 6 when energised, i.e. when supplied with current through a cable 7.

When the device is being used, the casing 5 is held so that the plate 6 will lie against the relevant part of the patient's body.

The apparatus also includes drive means for operating the light emitting device 1. The drive means is designed to control the light emitting device 1 to emit different monochromatic light of different wavelengths over different predetermined time periods, and to pulsate the light emitted in accordance with a predetermined pulse frequency or series of pulse frequencies over said time periods.

The light emitting device 1 may include light emitting diodes 3 which emit infrared light. These diodes are shown with solid circles in Figure 1. Visible light can be emitted with the aid of other light emitting diodes 4. These diodes are illustrated with empty circles in Figure 1. The infrared light diodes 3 will preferably be semi-conductors of the GaAs-type (Gallium Arsenide). The diodes 4 that emit visible light will also preferably be of the GaAs-type.

The drive means includes a computer 8 which controls drive circuits to which signals for driving or operating the light emitting diodes are sent from the computer via a conductor.

The cable 7, which includes the conductor, may for instance have a length of from one to three metres and extends between the drive means and the light emitting device 1. The drive means will have a size that corresponds generally to the size of a standard personal computer.

The computer and the drive circuits are of a suitable known kind. The drive means or computer has connected thereto a keyboard 13 by means of which the operator can key-in data for causing the drive means to activate the light emitting device in a desired manner. The apparatus will conveniently also include a display 14 for displaying the settings entered through the keyboard. This display may be the computer screen.

The light emitting device 1 includes light emitting diodes 4 which are adapted to emit essentially monochromatic visible light in one of the colours violet, blue, yellow, orange, red or green, and also infrared light and other invisible wavelengths.

The nature of the light used will depend on the disease or the type of injury to be treated.

A large part of the above description of the drawings is also found to a great part in the aforementioned patent specification.

According to the present invention at least the drive circuits 9, 10 of the drive means are mounted in the light emitting device 1. This means that a separate and specially adapted drive means of the aforedescribed kind is not required but can be replaced with other means.

The computer 8 is a personal computer and is free-standing in relation to the light emitting device. The computer is programmed to deliver a coded signal to a microprocessor 12 in the light emitting device via a conductor 11, said microprocessor functioning to activate or energise the drive circuits 9, 10 subsequent to decoding the signal; see Figure 3.

In this embodiment of the invention, the computer, which may be any type of commercially available PC, is programmed with a program that controls the computer to send to the microprocessor 12 coded signals that correspond to the treatment to be administered. The microprocessor 12 includes a memory and is permanently programmed to decode the coded signals and therewith activate the drive circuits 9, 10 which, in turn, activate the light emitting diodes 3, 4 in accordance with the treatment desired. The coded signals are transmitted to the light emitting device 1 through a cable 7. Electric current is supplied to the light emitting device 1 through a conductor 20 connected to a battery eliminator 19, to which a mains voltage is applied.

In this way, the necessary specific equipment has been reduced to include the light emitting device and the electronics incorporated therein, and a computer program that shall be read into a computer.

Figure 4 illustrates a second embodiment of the invention in which the computer 8 is integrated with the light emitting device 1. The computer 8 of this embodiment will conveniently have the form of a microprocessor and associated memory. The computer 8 is programmed to activate the light emitting diodes via the drive circuits 9, 10, such as to carry out different treatments. The various treatments desired can be fed into the computer via a suitable input means. The input means 15 of the computer 8 is disposed in the light emitting device. As illustrated in Figure 5, a preferred method is to arrange a known, simple keypad 16 and associated display 17 on the light emitting device 1.

According to this embodiment it is achieved that all of the equipment is accommodated in the light emitting device, wherewith said device need only be supplied with current via a conductor 18 and a battery eliminator 19.

It will be obvious that the present equipment is both cheaper and less bulky than the known equipment. This enables the equipment to be moved more easily between different treatment stations, among other things.

Although the invention has been described with reference to a number of exemplifying embodiments, it will be understood that the invention can be varied in many ways. For instance, the light emitting device can be powered through the same lead as the cable 7. The apparatus may also include more than two drive circuits. Moreover, the drive means may be designed to effect solely one given type of treatment, by emitting one or more types of monochromatic light over given time periods and with given pulse repetition frequencies.

The present invention is therefore not limited to the aforedescribed embodiments, since variations can be made within the scope of the following Claims.

## Claims

1. Apparatus for controlling external medical treatment administered with the aid of light, said apparatus including a light-emitting device (1) which is intended to be held against or in the close proximity of the patient's body, and means (8, 9, 10) for driving the light-emitting device, wherein the light-emitting device includes light-emitting diodes adapted to emit monochromatic light, wherein the drive means (8, 9, 10) is adapted to cause the light-emitting device (1) to emit one or more types of monochromatic light over one or more predetermined time periods and to pulsate the light emitted in accordance with a predetermined pulse frequency or series of pulse frequencies over said time periods, wherein said drive means (8, 9, 10) includes a computer (8) and circuits (9, 10) for the light-emitting diodes (3, 4), wherein the computer (8) includes input means (13,16) for inputting data relating to an intended treatment, wherein the computer (8) is adapted to deliver electric signals to the drive circuits (9, 10), wherewith intended light-emitting diodes (3, 4) function to emit light within predetermined time periods and at predetermined pulse repetition frequencies, **characterised in that** at least the drive circuits (9, 10) of the drive means are mounted in the light emitting device (1), **in that** the computer (8) is adapted to send a coded signal to a microprocessor (12) in the light emitting device (1) via a conductor (7), wherewith the microprocessor (12) functions to activate said drive circuits (9, 10) subsequent to decoding said signal and **in that** the computer (8) is a personal computer and is free-standing in relation to the light emitting device (1).

## Patentansprüche

1. Vorrichtung zur Steuerung einer äußeren medizinischen Behandlung, welche mit Hilfe von Licht ausgeführt wird, wobei die Vorrichtung eine Licht emittierende Vorrichtung (1) aufweist, die dafür vorgesehen ist, an oder in nächster Nähe des Körpers eines Patienten gehalten zu werden, und mit einer Einrichtung (8, 9, 10) zum Antreiben der Licht emittierenden Vorrichtung, wobei die Licht emittierende Vorrichtung Licht emittierende Dioden aufweist, die geeignet sind, monochromes Licht zu emittieren, wobei die Antriebseinrichtung (8, 9, 10) geeignet ist, die Licht emittierende Vorrichtung (1) zu veranlassen, einen oder mehrere Typen von monochromem Licht über eine oder mehrere vorbestimmte Zeitbereiche zu emittieren und das emittierte Licht gemäß einer vorbestimmten Pulsfrequenz oder Serien von Pulsfrequenzen über die Zeitbereiche zu pulsieren, wobei die Antriebseinrichtung (8, 9, 10) einen Computer (8) und Schaltungen (9, 10) für die Licht emittierenden Dioden (3, 4) aufweist, wobei der Computer (8) eine Eingabe-Einrichtung (13, 16) zum Eingeben von Daten entsprechend einer vorgesehenen Behandlung aufweist, wobei der Computer (8) geeignet ist, elektrische Signale zu den Antriebsschaltungen (9, 10) zu liefern, wodurch Licht emittierende Dioden (3, 4) derart funktionieren, dass sie Licht in vorbestimmten Zeitbereichen und in vorbestimmten Pulswiederholungsfrequenzen zu emittieren, **dadurch gekennzeichnet, dass** wenigstens die Antriebseinrichtungen (9, 10) der Antriebseinrichtungen in der Licht emittierenden Vorrichtung (1) angeordnet sind, dass der Computer (8) geeignet ist, ein kodiertes Signal zu einem Mikroprozessor (12) in der Licht emittierenden Vorrichtung (1) über einen Leiter (7) zu senden, womit der Mikroprozessor (12) so funktioniert, dass er die Antriebsschaltungen (9, 10) in Folge antreibt, um das Signal zu dekodieren, und dass der Computer (8) ein Personalcomputer ist und gegenüber der Licht emittierenden Vorrichtung (1) ein freistehendes Gerät ist.

## Revendications

1. Appareil permettant de commander un traitement médical externe administré au moyen de lumière, ledit appareil incluant un dispositif électroluminescent (1) qui est destiné à être maintenu contre ou à proximité du corps du patient, et un moyen (8, 9, 10) destiné à la conduite du dispositif électroluminescent, dans lequel le dispositif électroluminescent comprend des diodes électroluminescentes adaptées à émettre une lumière monochromatique, le moyen de conduite (8, 9, 10) est adapté à permettre l'émission par le dispositif électroluminescent (1) d'un ou plusieurs types de lumière monochromatique au cours d'une ou plusieurs périodes prédéterminées et à pulser la lumière émise en fonction d'une fréquence d'impulsions ou d'une série de fréquences d'impulsions prédéterminées au cours desdites périodes, le moyen de commande (8, 9, 10) comprenant un ordinateur (8) et des circuits (9, 10) destinés aux diodes électroluminescentes (3, 4), l'ordinateur (8) incluant un moyen d'entrée (13, 16) destiné à entrer des données relatives à un traitement prévu, selon lequel l'ordinateur (8) est adapté à appliquer des signaux électriques aux circuits de conduite (9, 10), grâce à quoi les diodes électroluminescentes prévues (3, 4) assurent par conséquent l'émission de lumière au cours des périodes prédéterminées et selon des fréquences de répétition d'impulsions prédéterminées,
**caractérisé en ce qu'**
au moins les circuits de conduite (9, 10) du moyen de conduite sont montés dans le dispositif électroluminescent (1), l'ordinateur (8) est adapté à envoyer un signal codé à un microprocesseur (12) dans le dispositif électroluminescent (1) via un conducteur (7), grâce à quoi le microprocesseur (12) assure par conséquent l'activation des circuits de conduite (9, 10) consécutive au décodage du signal, et l'ordinateur (8) est un ordinateur personnel, indépendant dans sa relation avec le dispositif électroluminescent (1).
